# FASCICULE DE BREVET EUROPEEN

(11) **EP 0 699 420 B1**
(45) Date de publication et mention de la délivrance du brevet: **09.10.2002**
(21) Numéro de dépôt: 95440042.0
(22) Date de dépôt: 05.07.1995
(51) Int. Cl.: A61B 17/86

(54) **Dispositif de cheville ostéosynthétique**
Osteosynthetische Stiftvorrichtung
Osteosynthetic pin device

(30) Priorité: 04.07.1994 FR 9408373
(43) Date de publication de la demande: 06.03.1996
(73) Titulaire: Depuy France, 69100 Villeurbanne (FR); PIED INNOVATION, 69003 Lyon (FR)
(72) Inventeur: Benichou, Michel, 34000 Montpellier (FR); Augoyard, Marc, 69160 Tassin La Demi-Lune (FR); Augagneur, Christian, 69007 Lyon (FR)
(74) Mandataire: Polus, Camille

(56) Documents cités:
- EP-A- 0 323 429
- WO-A-93/21848
- WO-A-93/23679
- DE-U- 8 631 649
- US-A- 4 537 185
- DATABASE WPI Week 8801 Derwent Publications Ltd., London, GB; AN 88-004047 & JP-A-62 268 553 (MITSUBISHI MINING) , 21 Novembre 1987

## Description

La présente invention a pour objet un dispositif de cheville ou vis de blocage pour plaque d'ostéosynthèse ou coaptation de deux fragments osseux.

On connaît déjà des vis autocassables, notamment celle décrite dans le document FR-A-8718540 (ou EP-A-0 323 429), qui comporte une tige filetée prolongée d'une tête hexagonale solidaire, par l'intermédiaire d'un étranglement de moindre résistance à un couple donné, d'un embout d'adaptation dans le mandrin d'un outil électrique de vissage: le chirurgien peut ainsi visser directement et rapidement avec l'outil de vissage ce qui permet de réduire les temps opératoires.

Toutefois ce type de vis présente plusieurs inconvénients, en effet la tête de la vis déborde la plaque d'ostéosynthèse ce qui peut constituer une gêne. D'autre part on utilise habituellement pour la fixation de plaques d'ostéosynthèse des vis à tête fraisée à empreinte hexagonale en creux, mais du fait de la solidarisation à l'embout de maintien il n'est pas possible de munir la tête d'une empreinte en creux, alors qu'il est nécessaire de pouvoir manoeuvrer manuellement la vis afin soit de finir le vissage soit de la dévisser pour l'enlever au bout d'un certain temps.

De plus dans la mise en oeuvre de ce type de vis le forage est réalisé par l'intermédiaire de la pointe de la vis et d'une entaille latérale pratiquée non loin de ladite pointe. Or ce type d'entaille, qui sert essentiellement au taraudage, ne peut permettre de forer, en sorte que la pénétration de la vis est faite en force plus par percussion que par enlèvement de copeaux, au risque d'une mauvaise réalisation du taraudage et donc d'une solidarisation approximative de la vis dans son support.

La présente invention a pour objet un dispositif de cheville ou vis sécable auto-foreuse et auto-taraudeuse permettant de remédier à ces inconvénients.

L'invention a donc pour objet un dispositif de cheville auto-foreuse et auto-taraudeuse, comprenant un embout sécable et une partie cheville avec une tige filetée se terminant par une extrémité perforante auto-foreuse et possédant une tête solidaire de l'embout par une zone de moindre résistance , pour le blocage d'une plaque d'ostéosynthèse ou la coaptation de deux fragments osseux, caractérisé en ce que l'extrémité auto-foreuse de la tige filetée est un plat positionné diamétralement suivant une allure de pointe constituée de deux pans, qui forment un angle obtus, et qui sont biseautés de façon symétrique, et en ce que la tête est fraisée et comporte périphériquement, diamétralement opposées, deux entailles constituant une empreinte femelle qui permet la préhension de ladite cheville au moyen d'un outil d'empreinte mâle correspondante.

Selon une caractéristique additionnelle du dispositif selon l'invention, la cheville comporte une partie lisse entre la tige filetée et la base de la tête afin de permettre en fin de vissage de comprimer la plaque d'ostéosynthèse ou la corticale proximale .

Selon une autre caractéristique additionnelle du dispositif selon l'invention, la cheville comporte un orifice axial permettant le passage d'une broche de guidage.

Une cheville selon l'invention permet ainsi de réaliser, en un seul mouvement, la perforation et le taraudage, avec enfouissement presque total de la tête dans la plaque d'ostéosynthèse, ou dans l'os, son enlêvement ultérieur demeurant néanmoins possible.

Dans le cas d'une cheville perforée, le plat de l'extrémité foreuse ne comporte pas de pointe, mais seulement des portions de pans obliques biseautés, qui permettent d'aléser l'orifice pré-percé pour l'introduction de la broche.

Le taraudage est réalisé de deux manières selon la taille de la cheville. Ainsi pour une cheville relativement importante, une entaille latérale est pratiquée à l'extrémité du filet, tandis que pour une cheville de petite taille, la finesse du filet permet de se passer de taraudage.

Les avantages et les caractéristiques de la présente invention ressortiront plus clairement de la description qui suit et qui se rapporte au dessin annexé, lequel en représente un mode de réalisation non limitatif.

Dans le dessin annexé :
- la figure 1 représente une vue en plan d'un dispositif de cheville selon un mode préféré de l'invention.
- la figure 2 représente une vue en plan de face de l'extrémité perforante du même dispositif.
- la figure 3 représente une vue en coupe selon l'axe XX' de la figure 1.

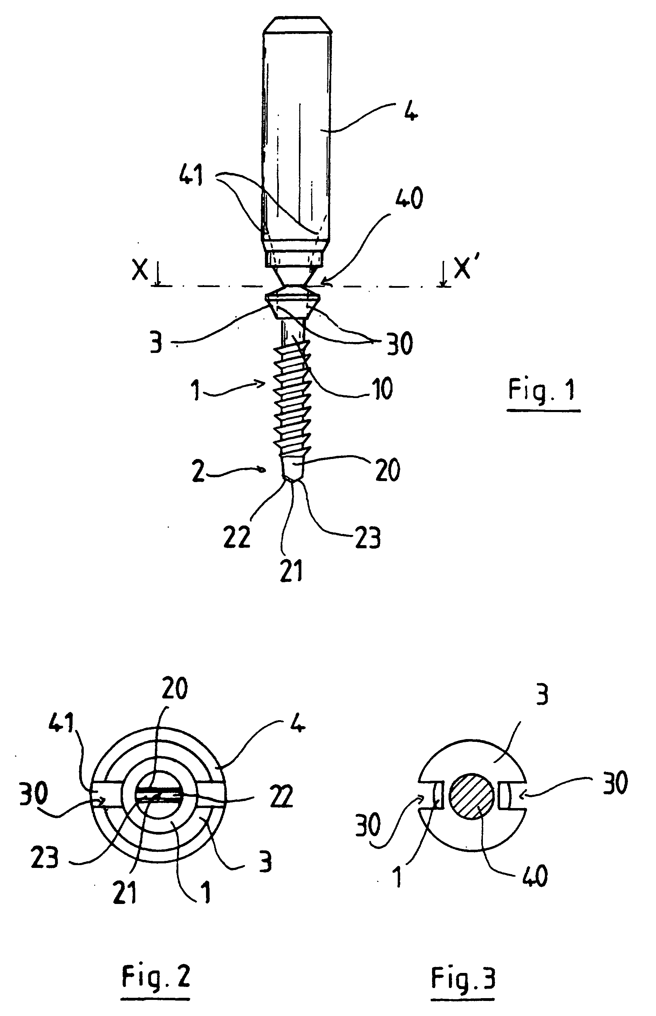

Si on se réfère à la figure 1 on peut voir qu'un dispositif de cheville selon un mode préféré de l'invention comprend une partie cheville avec une tige filetée 1 prolongée d'un côté par une extrémité perforante 2, et de l'autre par une partie intermédiaire 10 non filetée, et une tête fraisée 3 solidarisée, par l'intermédiaire d'une zone 40 de moindre résistance à un couple donné, à un embout 4 d'adaptation dans le mandrin d'un outil électrique ou pneumatique.

Lors du serrage de la cheville, la plaque d'ostéosynthèse, ou la corticale proximale dans le cas d'une coaptation, se positionne autour de la partie intermédiaire 10 entre la tête 3 et la tige filetée 1, ce qui permet un meilleur serrage.

Il est à noter que l'embout 4 peut comporter des éléments permettant son adaptation par encliquetage sur un mandrin adéquat, ne nécessitant pas d'opération de serrage.

Si on se réfère également à la figure 2 on peut voir que l'extrémité perforante 2 est constituée d'un plat 20 qui est positionné diamétralement par rapport à la tige filetée 1, et dont l'extrémité est découpée en pointe 21, les deux pans 22 et 23 de cette pointe 21 faisant un angle obtus, approximativement de 120°, et étant biseautés symétriquement l'un par rapport à l'autre de manière à créer des. bords d'attaque tranchants.

Si on se réfère maintenant à la figure 3 on peut voir que la tête fraisée 3 comporte deux entailles 30 diamétralement opposées permettant d'y accoupler un outil, non représenté, d'empreinte mâle adaptée, en vue du dévissage de la cheville, et permettant aussi, le cas échéant, de finir le vissage manuellement.

Les entailles 30 sont réalisées par meulage ou fraisage, et on peut voir sur la figure 1 que les traits de meule 41 se prolongent dans l'embout 4, en sorte qu'en prolongeant le meulage tout le long de l'embout 4 on pourrait pratiquer deux rainures longitudinales diamétralement opposées permettant l'encliquetage de l'embout 4, par l'intermédiaire d'un adaptateur ou non, dans un mandrin de profil adéquat.

L'ensemble du dispositif selon l'invention, ou tout au moins la partie cheville, peut être réalisé en n'importe quel matériau métallique biocompatible, par exemple du titane ou du TA 6 V, un alliage chrome-cobalt ou un acier inox, mais peut l'être aussi en un matériau biodégradable et biocompatible, ou en un matériau minéral biointégrable, par exemple le phosphate de calcium, ou l'hydroxyapatite.

A cet égard il convient de souligner l'importance d'une tête fraisée enfouissable, dans le cas d'une coaptation au moyen d'une cheville réalisée en matériau biointégrable, et ne nécessitant pas d'être enlevée.

## Revendications

1. Dispositif de cheville auto-foreuse et auto-taraudeuse, comprenant un embout sécable (4) et une partie cheville avec une tige filetée (1) se terminant par une extrémité perforante auto-foreuse (2) et possédant une tête (3) solidaire de l'embout (4) par une zone de moindre résistance (40), pour le blocage d'une plaque d'ostéosynthèse ou la coaptation de deux fragments osseux, **caractérisé en ce que** l'extrémité auto-foreuse (2) de la tige filetée (1) est un plat (20) positionné diamétralement suivant une allure de pointe (21) constituée de deux pans (22, 23), qui forment un angle obtus, et qui sont biseautés de façon symétrique, et **en ce que** la tête (3) est fraisée et comporte périphériquement, diamétralement opposées, deux entailles (30) constituant une empreinte femelle qui permet la préhension de ladite cheville au moyen d'un outil d'empreinte mâle correspondante.

2. Dispositif selon la revendication 1 **caractérisé en ce qu'**il comporte une partie lisse (10) entre la tige filetée (1) et la tête (3).

3. Dispositif selon la revendication 1 ou la revendication 2 **caractérisé en ce qu'**il comporte un orifice axial permettant le passage d'une broche de guidage.

4. Dispositif selon l'une quelconque des revendications précédentes **caractérisé en ce que** l'extrémité de la tige filetée (1) comporte une entaille de taraudage.

5. Dispositif selon l'une quelconque des revendications précédentes **caractérisé en ce que** les entailles (30) de la tête (3) sont réalisées par meulage ou fraisage latéral et vertical.

6. Dispositif selon l'une quelconque des revendications précédentes **caractérisé en ce que** l'embout (4) comporte des éléments d'adaptation par encliquetage dans un mandrin adéquat.

7. Dispositif selon la revendication 6 **caractérisé en ce que** les entailles (30) de la tête (3) sont prolongées dans l'embout (4) afin de permettre l'adaptation de celui-ci dans un mandrin de profil correspondant.

8. Dispositif selon l'une quelconque des revendications précédentes **caractérisé en ce qu'**au moins la partie cheville est réalisée en un matériau biodégradable et biocompatible.

9. Dispositif selon l'une quelconque des revendications de 1 à 7 **caractérisé en ce que** qu'au moins la partie cheville est réalisée en un matériau biointégrable.

## Claims

1. Self-tapping, self-drill anchor device, comprising a divisible endpiece (4) and an anchor portion with a threaded rod (1) terminating in a self-drill drilling end (2) and having a head (3) which is firmly attached to the endpiece (4) via a zone of reduced resistance (40), for securing an osteosynthesis plate or bringing together two bone fragments, **characterised in that** the self-drill end (2) of the threaded rod (1) is a flat part (20) positioned diametrically in the direction of a tip (21) consisting of two sections (22, 23) which form an obtuse angle and are bevelled symmetrically, and **in that** the head (3) is milled and has, in diametrically opposite positions on its periphery, two notches (30) constituting a female indentation enabling the anchor to be gripped by a tool with a corresponding male shape.

2. Device according to claim 1, **characterised in that** it comprises a smooth part (10) between the threaded rod (1) and the head (3).

3. Device according to claim 1 or 2, **characterised in that** it comprises an axial opening allowing a guide spindle to pass through.

4. Device according to any one of the preceding claims, **characterised in that** the end of the threaded rod (1) comprises a tapping notch.

5. Device according to any one of the preceding claims, **characterised in that** the notches (30) in the head (3) are produced by lateral and vertical grinding or milling.

6. Device according to any one of the preceding claims, **characterised in that** the endpiece (4) comprises adapting means designed to engage in an appropriate mandrel.

7. Device according to claim 6, **characterised in that** the notches (30) in the head (3) are extended into the endpiece (4) to allow said endpiece to be fitted in a mandrel of corresponding profile.

8. Device according to any one of the preceding claims, **characterised in that** at least the anchor part is made of a biodegradable and biocompatible material.

9. Device according to any one of claims 1 to 7, **characterised in that** at least the anchor part is made of a biointegrable material.

## Patentansprüche

1. Selbstbohrende und selbst gewindeschneidende Stiftvorrichtung, umfassend ein teilbares Ansatzstück (4) und einen Stiftteil mit einem gewindetragenden Schaft (1), dessen äußeres, durchbohrendes Ende von einem Selbstbohrer (2) abgeschlossen wird und einen über das Ansatzstück (4) durch eine Sollbruchstelle (40) verbundenen Kopf (3) zur Arretierung einer Platte zur Osteosynthese oder der Verbindung von zwei Knochenfragmenten, **dadurch gekennzeichnet, dass** das äußere, selbstbohrende Ende (2) des gewindetragenden Schaftes (1) eine diametral angeordnete Fläche (20) ist, die in eine Spitze (21) mündet, bestehend aus zwei Seiten (22, 23), die einen stumpfen Winkel formen, wobei diese symmetrisch, von abgekanteter Gestalt sind, und dass der Kopf (3) gefräst ist, wobei auf seinem Umfang diametral entgegengesetzt zwei Einkerbungen (30) weiblicher Prägung angeordnet sind, die das Greifen des besagten Stiftes mit Hilfe eines Werkzeugs entsprechender männlicher Prägung erlauben.

2. Vorrichtung nach Anspruch 1, **dadurch gekennzeichnet, dass** diese einen geglätteten Abschnitt (10) zwischen dem gewindetragenden Schaft (1) und dem Kopf (3) umfasst.

3. Vorrichtung nach einem der Ansprüche 1 oder 2, **dadurch gekennzeichnet, dass** diese eine axiale Öffnung umfasst, die den Durchgang einer Führungsspindel erlaubt.

4. Vorrichtung nach einem der Ansprüche 1 bis 3, **dadurch gekennzeichnet, dass** das äußere Ende des gewindetragenden Schaftes (1) eine schraubenförmige Einkerbung umfasst.

5. Vorrichtung nach einem der Ansprüche 1 bis 4, **dadurch gekennzeichnet, dass** die Einkerbungen (30) des Kopfes (3) durch seitliches und vertikales Schleifen oder Fräsen erzeugt werden.

6. Vorrichtung nach einem der Ansprüche 1 bis 5, **dadurch gekennzeichnet, dass** das Ansatzstück (4) Adaptionselemente zum Einrasten in einem entsprechenden Spannfutter umfasst.

7. Vorrichtung nach Anspruch 6, **dadurch gekennzeichnet, dass** die Einkerbungen (30) des Kopfes (3) Fortführungen des Ansatzstücks (4) sind, um die Anpassung derselben in einem Spannfutter mit übereinstimmendem Profil zu erlauben.

8. Vorrichtung nach einem der Ansprüche 1 bis 7, **dadurch gekennzeichnet, dass** zumindest ein Teil des Stiftes aus biologisch abbaubarem und biologisch verträglichem Material hergestellt ist.

9. Vorrichtung nach einem der Ansprüche 1 bis 7, **dadurch gekennzeichnet, dass** zumindest ein Teil des Stiftes aus biologisch integrierbarem Material hergestellt ist.
